(19) Europäisches Patentamt
European Patent Office
Office européen des brevets

(11) Publication number: **0 456 514 B1**

(12) # EUROPEAN PATENT SPECIFICATION

(45) Date of publication of patent specification: **19.10.94**

(51) Int. Cl.⁵: **C07D 473/40**, C07D 473/00, A61K 31/52

(21) Application number: **91304235.4**

(22) Date of filing: **10.05.91**

(54) **2-Fluoroneplanocin A and its production.**

(30) Priority: **11.05.90 JP 122177/90**

(43) Date of publication of application:
**13.11.91 Bulletin 91/46**

(45) Publication of the grant of the patent:
**19.10.94 Bulletin 94/42**

(84) Designated Contracting States:
**AT BE CH DE DK ES FR GB GR IT LI LU NL SE**

(56) References cited:
**EP-A- 0 304 889**
**EP-A- 0 334 361**
**EP-A- 0 335 355**
**EP-A- 0 347 852**

**CANCER RESEARCH, vol. 46, no. 3, 1986, pages 1063-1067; M. INABA et al.: "Biochemical Mode of Cytotoxic Action of Neplanocin A in L1210 Leukemic Cells"**

(73) Proprietor: **ASAHI KASEI KOGYO KABUSHIKI KAISHA**
**2-6, Dojimahama 1-chome**
**Kita-ku**
**Osaka (JP)**

(72) Inventor: **Shuto, Satoshi**
**690-10, Makinokou,**
**Shuzenji-cho**
**Tagata-gun, Shizuoka (JP)**
Inventor: **Obara, Takumi**
**59-1, Ohdoi,**
**Kannami-cho**
**Tagata-gun, Shizuoka (JP)**
Inventor: **Fujiwara, Tatsuro**
**310, Nirayama,**
**Nirayama-cho**
**Tagata-gun, Shizuoka (JP)**
Inventor: **Itoh, Hiromichi**
**38-2, Sankeidai**
**Mishima-shi, Shizuoka (JP)**

(74) Representative: **Woods, Geoffrey Corlett et al**
**J.A. KEMP & CO.**
**14 South Square**
**Gray's Inn**
**London WC1R 5LX (GB)**

EP 0 456 514 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 98, no. 21, 23rd May 1983 Columbus, Ohio, USA E.N. SPREMULLI et al.: "Hemodynamic effects of potentially useful antineoplastic agents" page 50; ref. no. 172756R & JNCI, J. Natl. Cancer Inst. 1983, 70 (3), 499-504

CHEMICAL ABSTRACTS, vol. 99, no. 23, 5th December 1983 Columbus, Ohio, USA V.I. AVRAMIS et al.: "Metabolism of 9-beta-D-arabinosyl-2-fluoroadenine-5'-phosphate by mice bearing P388 leukemia" page 22; ref. no. 187044B & Cancer Drug Delivery 1983, 1(1), 1-10

JOURNAL OF THE AMERICAN CHEMICAL SOCIETY, vol. 82, no. 2, 1960, pages 463-468; J.A. MONTGOMERY et al.: "Synthesis of potential anticancer agents. XX. 2-fluoropurines"

## Description

The present invention relates to a neplanocin A derivative and pharmacologically acceptable salts thereof having anti-neoplastic activity.

Neplanocin A is a compound of formula:

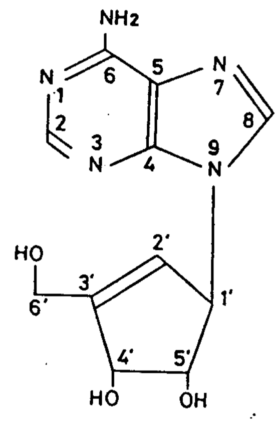

Neplanocin A has cytotoxic activity in vitro (Proceeding of 11th International Congress of Chemotherapy, Vol. 2, 1559-1561 (1979)) (hereinafter designated as ref. 1). However its cytotoxic activity is not sufficient for use as an anti-neoplastic agent. Derivatives of neplanocin A have also been synthesized (JP-A-57-163383, JP-A-57-102889, JP-A-58-85898, JP-A-58-118586, JP-A-58-183691, JP-A-59-219284 and JP-A-2-40369). However, no derivatives superior to neplanocin A have been discovered.

The cytotoxic activity of neplanocin A was disclosed after phosphorilation of the hydroxyl at the 6′-position by kinase in cells (Cancer Res., 46: 1063-1067 (1986)) (hereinafter designated as ref. 2). It is therefore thought that neplanocin A has no action on cells having no kinase activity for neplanocin A as a substrate. Furthermore the cytotoxic activity of neplanocin A in vivo is thought to be reduced by the action of adenocine deaminase, which is widely distributed in vivo, with rapid convertion to an inert deamination-type compound (neplanocin D) (ref. 1).

Therefore derivatives of neplanocin A which are not inactivated by adenosine deaminase are highly desirable.

We have surprisingly found that 2-fluoroneplanocin A of formula

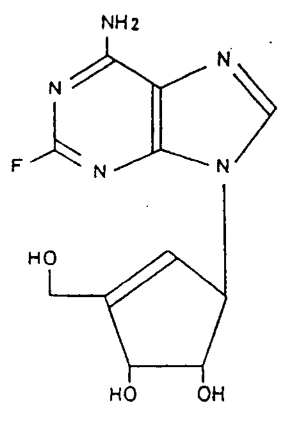

is not inactivated by adenosine deaminase and has a higher level of cytotoxic activity than that of neplanocin A.

The present invention provides 2-fluoroneplanocin A or a pharmacologically acceptable salt thereof.

The present invention also provides a pharmaceutical composition which comprises 2-fluoroneplanocin A or a pharmacologically acceptable salt thereof and a pharmaceutically acceptable diluent.

The present invention further provides 2-fluoroneplanocin A or a pharmacologically acceptable salt thereof for use in a method of treatment of the human or animal body by therapy.

The present invention additionally provides 2-fluoroneplanocin A or a pharmacologically acceptable salt thereof for use as an anti-neoplastic agent.

The present invention yet further provides use of 2-fluoroneplanocin A or a pharmacologically acceptable salt thereof in the manufacture of a medicament for the treatment of a condition requiring an anti-neoplastic agent.

The pharmacologically acceptable salt is a nontoxic salt. Examples are inorganic salts such as hydrochloride, sulfate or phosphate and organic salts such as acetate, propionate, tartrate, citrate, glycolate, gluconate, succinate, malate, glutamate, aspartate or methanesulfonate. Other non-toxic salts with organic acid are also included.

2-fluoroneplanocin A can be obtained by reacting a compound of formula

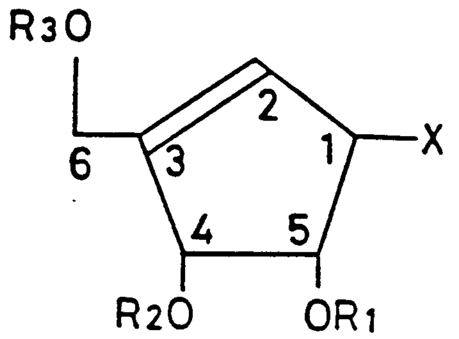

(1)

wherein $R_1$ and $R_2$ are each hydrogen or a hydroxy protecting group, $R_3$ is a hydroxy protecting group and X is a leaving group, with 2-fluoroadenine to produce a compound of formula

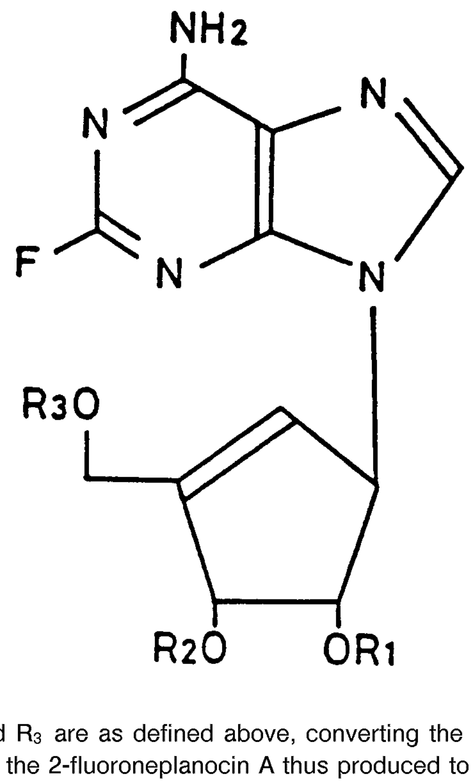

(2)

wherein $R_2$, $R_2$ and $R_3$ are as defined above, converting the hydroxy protecting groups to hydrogen and, if desired, converting the 2-fluoroneplanocin A thus produced to a pharmacologically acceptable salt thereof.

Hydroxy protecting groups at positions 4, 5 and 6 in the above compound (1) are known protecting groups used in nucleic acid chemistry. Examples at positions 4 and 5 are acyl groups such as formyl acetyl, methoxyacetyl, benzoyl or p-chlorobenzyloxyacetyl, ether-type protective groups such as t-butyl, benzyl, trityl, α-ethoxyethyl, α-methoxyisopropyl, tetrahydropyranyl, methoxytetrahydropyranyl, o-nitro-benzyl, or t-butyldiphenylsilyl. Further examples are a ketone compound residue in which a cyclic acetal is formed together with the two adjacent oxygen atoms at positions 4 and 5, for example isopropylidene, methoxymethylene, methoxyethylidene, ethoxymethylene, ethoxyethylene, benzylidene and cycloalkylidene. These protecting groups can be introduced by reaction with the corresponding aldehyde or ketone in the

present of an acid catalyst.

Examples of hydroxy protecting groups at position 6 are acyl groups such as formyl, acetyl, chloroacetyl, trichloroacetyl, trifluoroacetyl, pyvaloyl, benzoyl, β-benzoylpropionyl, phenoxyacetyl or trityloxyacetyl, ether-type protective groups such as trityl, monomethoxytrityl, dimethoxytrityl or trimethoxytrityl, methoxymethyl or benzyl.

Examples of the leaving group X are electron withdrawing groups such as methanesulfonyloxy, p-toluenesulfonyloxy or halogen. The compound of formula (1) wherein, for example, X is methanesulfonyloxy or p-toluenesulfonyloxy, is a known compound, and can be produced according to the method described in J. Org. Chem., 53 (24): 5707-5714 (1988).

2-fluoroadenine used in the present invention is a known compound and is described in J. Am. Chem. Soc., 82 (Jan. 20): 463-468 (1960).

The reaction of the compound of formula (1) with 2-fluoroadenine can be carried out in a polar solvent such as dimethylformamide dimethylsulfoxide, dimethylacetamide or acetonitrile in the present of a base such as $K_2CO_3$ and a crown ether. The molar ratio of the compound of formula (1) and 2-fluoroadenine is theoretically 1:1, but in general a 2-3 molar excess of 2-fluoroadenine is used. The reaction generally proceeds at from room temperature to 100 °C.

Isolation of the thus-obtained compound of formula (2) from the reaction mixture can be performed by removing the reaction solvent, adding a solvent such as ethyl acetate, removing the insoluble material and concentrating the filtrate in vacuo. Further purification can be effected by column chromatography using an adsorption agent such as silica-gel.

2-fluoroneplanocin A can be obtained by removing the hydroxy protecting group(s) from the compound of formula (2) by known removal techniques used in nucleic acid chemistry. For example, in the case that protecting groups at positions 4' and 5' are isopropylidene and the protecting group at position 6' is benzyl, the group at position 6' is benzyl, the groups can be removed by treatment with a Lewis acid such as $BCl_3$ or $BBr_3$ in an inert solvent such as dichloromethane under cooling conditions.

Isolation and purification of the thus obtained 2-fluoroneplanocin A from the reaction mixture can be effected by removing the reaction solvent, dissolving the residue in a solvent such as methanol, and subjecting this solution to column chromatography using silica-gel with an elution solvent such as chloroform-methanol.

A pharmacologically acceptable salt of the thus-obtained 2-fluoroneplanocin A can be prepared, if desired, by any known method.

No death is observed for a single administration of 2-fluoroneplanocin A, 10 mg/kg, intraperitoneally in mice. 2-fluoroneplanocin A can be applied as an anti-neoplastic agent. The dose can be determined by considering the curative effect, route of administration, age or body weight of a patient. In general 10-500 mg/man/day is used. Administration can be carried out orally or parenterally, for example by intravenous injection, drop infusion or intramuscular administration.

The growth inhibition activity of L5178Y cells and the resistance to adenosine deaminase of 2-fluoroneplanocin A are illustrated hereinbelow:

(1) Growth inhibition activity against L5178Y cells:

A solution of each test compound is prepared by dissolving the compound in RRMI 1640 medium containing 10% bovine serum. A cell suspension of L5178Y cells, $1.1 \times 10^4$ cells/ml, each suspension having a volume of 1.8 ml, is added to each test tube. A solution (0.2 ml) of the test compound is added thereto and the medium is tightly sealed and incubated at 37 °C for 48 hours. After incubation, the number of cells is measured using a Coulter counter and the growth ratio of the cells is determined.

The result is shown in Table 1.

Table 1

| Growth inhibition against L5178Y cells | |
|---|---|
| Test sample | $IC_{50}$ (μg/ml) |
| Reference (neplanocin A) | 0.2 |
| 2-fluoroneplanocin A | 0.06 |

(2) Resistance to adenosine deaminase:

A solution (0.5 mM) of each test sample is prepared by dissolving the test sample in 0.05 M Tris-HCl buffer (pH 7.2). Adenosine deaminase (calf intestine, 400 U/ml glycerin, Boehringer 102091) is diluted ten times with 0.05 M Tris-HCl buffer (pH 7.2) to prepare an adenosine deaminase solution.

0.5 ml of each sample solution is mixed with 10 µl of adenosine deaminase solution. The mixtures are allowed to stand in a water bath at 25 °C, and 4 µl samples thereof are pipetted off after 0 and 30 minutes. The remaining sample is measured by HPLC under the following conditions:

| HPLC | |
|---|---|
| Column | Supersher RP-18-4 |
| Eluent | 15% methanol |
| Flow rate | 1.0 ml/min |
| Temperature | 50 °C |

The result is shown in Table 2.

Table 2

| Test Sample | Remaining ratio (%) | |
|---|---|---|
| | 0 min | 30 min |
| Reference (neplanocin A) | 100 | 0 |
| 2-fluoroneplanocin A | 100 | 100 |

As shown by the above, 2-fluoroneplanocin A has growth inhibitory activity against L5178Y cells of three times higher that of known neplanocin A. Moreover the compound is not decomposed by adenosine deaminase even after 30 minutes of incubation and hence it has a very high resistance, which is much greater than that of neplanocin A.

The following Examples further illustrate the present invention.

Example 1

Production of (-) -9-[(1R,4R,5S)-3-[(benzyloxy) methyl]-4,5-0-isopropylidene-2-cyclopentene-1-yl]-2-fluoroadenine:

2-fluoroadenine (427 mg, 2.79 m mol), $K_2CO_3$ (386 mg, 2.79 m mol) and 16-crown-6 (369 mg, 1.4 m mol) were mixed with stirring in dimethylformamide (30 ml) under an argon atmosphere at 75 °C. (1S, 4R, 5S)-(-)-3-[(benzyloxy)methyl]-4,5-0-isopropylidenedioxy-2-cyclopentene-1-ol•p-toluenesulfonate (600 mg, 1.4 m mol) was added to the reaction mixture and stirred at 75 °C for 1 hour. Dimethylformamide was removed in vacuo and ethyl acetate (50 ml) was added to the residue. Insoluble material was removed and the filtrate was washed with saturated sodium chloride solution. The filtrate was passed through Whatman-IPS filter paper and concentrated in vacuo. The residue was purified by column chromatography using silica-gel [(Merck, Art 9385, 20 g) flash column eluted with chloroform-methanol (100:1)] to obtain the product (172 mg, yield 30%), which was crystallized from hexanethyl acetate as needle crystals.

$^1$H-NMR($CDCl_3$)δ;

1. 36、1.47 (each s、each 3H, $CH_3$)、
4. 28 (bs、2H、H-6' a, b)、
4. 61 (abq、J = 12. OHz、2H、$OCH_2$ $C_6$ $H_5$)、
4. 73 (d、J = 5. 6Hz、1H、H-5')、
5. 41 (d、J = 5. 6HZ、1H、、H-4')、
5. 48 (bs、1H H-1')、
5. 78 (bs、2H、$NH_2$)、
7. 83 (m、5H、phenyl)
7. 64 (s、1H、H-8)、

FAB Mass; 412 ($MH^+$ )
Rf; 0.46 (chloroform - methano = 10:1、Merck Art 5715 plate)

Example 2

Production of 2-fluoroneplanocin A:

A solution of $BCl_3$ (1M) in hexane (2.7 ml) was added to a solution of (-)-9-[(1R,4R,5S)[(benzyloxy)-methyl)-4,5-0-isopropylidenedioxy-2-cyclopentene-1-yl]-2-fluoroadenine (150 mg, 0.365 m mol) obtained in Example 1 in dichloromethane (15 ml) at -75 ° C under an argon atmosphere with stirring, and further stirred at -75 ° C for 3 hours. Methanol (3 ml) was added to the reaction mixture and concentrated in vacuo. Methanol was further added to the reaction mixture and concentrated in vacuo. The same operation was repeated three times, and the resulting methanol solution was adjusted to pH 10 by adding 7% aqueous ammonia and concentrated in vacuo. The residue was treated by silica-gel (Merck, Art 9835, 15 g) flash column chromatography eluted with chloroform-methanol-conc. aq. ammonia (200:10:0.5 → 200:40:2) to obtain crude 2-fluoroneplanocin A (41 mg).

The crude material was purified by preparative HPLC (column : LICHROSORB RP 18-5, 20 x 250 mm, elution : 10% methanol) to obtain pure crystals (23 mg).

$^1H$-NMR($CD_3$ OD)$\delta$;
4. 31 (bs、2H、H-6' a, b)、
4. 37 (t、J = 5. 4Hz、1H、H-5')、
4. 62 (d、J = 5. 4Hz、1H、H-4')、
5. 40 (m、1H、H-1')
5. 88 (d、J = 2Hz、H-2')、
8. 02 (s、1H、H-8)、
FAB Mass; 282 ($MH^+$)
Rf; 0.46 (chloroform - methano - water = 65:25:3、Merck Art 5715 plate)

**Claims**

**Claims for the following Contracting States : AT, BE, CH, DE, DK, FR, GB, IT, LI, LU, NL, SE**

1. 2-fluoroneplanocin A or a pharmacologically acceptable salt thereof.

2. 2-fluoroneplanocin A according to claim 1 wherein the salt is the hydrochloride, sulfate, phosphate, acetate, propionate, tartrate, citrate, glycolate, gluconate, succinate, malate, glutamate, aspartate or methanesulfonate.

3. A process for the production of 2-fluoroneplanocin A or a pharmacologically acceptable salt thereof as defined in claim 1 or 2, which process comprises reacting a compound of formula:

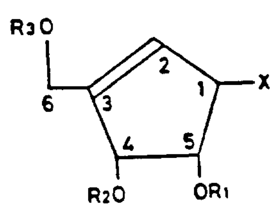

wherein $R_1$ and $R_2$ are each hydrogen or a hydroxy protecting group, $R_3$ is a hydroxy protecting group and X is a leaving group, with 2-fluoroadenine to produce a compound of formula:

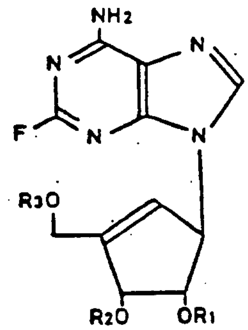

wherein $R_1$, $R_2$ and $R_3$ are as defined above, converting the hydroxy protecting groups to hydrogen and, if desired, converting the 2-fluoroneplanocin A thus produced to a pharmacologically acceptable salt thereof.

**4.** A process according to claim 3, wherein $R_1$ and $R_2$ are each formyl, acetyl, methoxyacetyl, benzoyl, p-chlorobenzyloxyacetyl, t-butyl, benzyl, trityl, α-ethoxyethyl, α-methoxyisopropyl, tetrahydropyranyl, methoxy-tetrahydropyranyl, o-nitro-benzyl or t-butyldiphenylsilyl groups, or wherein $R_1$ and $R_2$ together form an isopropylidene, methoxymethylene, methoxyethylidene, ethoxymethylene, ethoxyethylene, benzylidene or cycloalkylidene group.

**5.** A process according to claim 3 to 4, wherein $R_3$ is a formyl, acetyl, chloroacetyl, trichloroacetyl, trifluoroacetyl, pyvaloyl, benzoyl, β-benzoylpropionyl, phenoxyacetyl, trityloxyacetyl, trityl, monomethoxytrityl, dimethoxytrityl, trimethoxytrityl, monomethoxymethyl or benzyl group.

**6.** A process according to any one of claims 3 to 5, wherein X is a methanesulfonyloxy or p-toluenesulfonyloxy group or a halogen atom.

**7.** A pharmaceutical composition which comprises 2-fluoroneplanocin A or a pharmacologically acceptable salt thereof as defined in claim 1 or 2 and a pharmaceutically acceptable diluent.

**8.** 2-fluoroneplanocin A or a pharmacologically acceptable salt thereof as defined in claim 1 or 2 for use in a method of treatment of the human or animal body by therapy.

**9.** 2-fluoroneplanocin A or a pharmacologically acceptable salt thereof as defined in claim 1 to 2 for use as an anti-neoplastic agent.

**10.** Use of 2-fluoroneplanocin A or a pharmacologically acceptable salt thereof as defined in claim 1 to 2 in the manufacture of a medicament for the treatment of a condition requiring an anti-neoplastic agent.

**Claims for the following Contracting States : ES, GR**

**1.** A process for the production of 2-fluoroneplanocin A or a pharmacologically acceptable salt thereof which process comprises reacting a compound of formula:

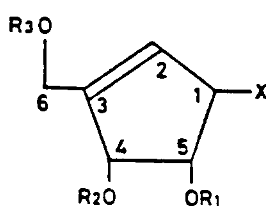

wherein $R_1$ and $R_2$ are each hydrogen or a hydroxy protecting group, $R_3$ is a hydroxy protecting group and X is a leaving group, with 2-fluoroadenine to produce a compound of formula:

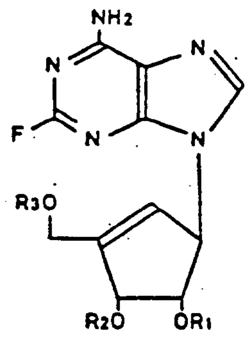

wherein $R_1$, $R_2$ and $R_3$ are as defined above, converting the hydroxy protecting groups to hydrogen and, if desired, converting the 2-fluoroneplanocin A thus produced to a pharmacologically acceptable salt thereof.

**2.** A process according to claim 1 wherein the salt is the hydrochloride, sulfate, phosphate, acetate, propionate, tartrate, citrate, glycolate, gluconate, succinate, malate, glutamate, aspartate or methanesulfonate.

**3.** A process according to claim 1 or 2, wherein $R_1$ and $R_2$ are each formyl, acetyl, methoxyacetyl, benzoyl, p-chlorobenzyloxyacetyl, t-butyl, benzyl, trityl, $\alpha$-ethoxyethyl, $\alpha$-methoxyisopropyl, tetrahydropyranyl, methoxy-tetrahydropyranyl, o-nitro-benzyl or t-butyldiphenylsilyl groups, or wherein $R_1$ and $R_2$ together form an isopropylidene, methoxymethylene, methoxyethylidene, ethoxymethylene, ethoxyethylene, benzylidene or cycloalkylidene group.

**4.** A process according to any one of the preceding claims, wherein $R_3$ is a formyl, acetyl, chloroacetyl, trichloroacetyl, trifluoroacetyl, pyvaloyl, benzoyl, $\beta$-benzoylpropionyl, phenoxyacetyl, trityloxyacetyl, trityl, monomethoxytrityl, dimethoxytrityl, trimethoxytrityl, monomethoxymethyl or benzyl group.

**5.** A process according to any one of the preceding claims, wherein X is a methanesulfonyloxy or p-toluenesulfonyloxy group or a halogen atom.

**6.** A process for preparing a pharmaceutical composition which comprises mixing together 2-fluoroneplanocin A or a pharmacologically acceptable salt thereof as defined in claim 1 or 2 and a pharmaceutically acceptable diluent.

**Patentansprüche**

**Patentansprüche für folgende Vertragsstaaten : AT, BE, CH, DE, DK, FR, GB, IT, LI, LU, NL, SE**

**1.** 2-Fluorneplanocin A oder eines seiner pharmakologisch verträglichen Salze.

**2.** 2-Fluorneplanocin A nach Anspruch 1, wobei das Salz das Hydrochlorid, Sulfat, Phosphat, Acetat, Propionat, Tartrat, Zitrat, Glykolat, Glukonat, Succinat, Malat, Glutamat, Aspartat oder Methansulfonat ist.

**3.** Verfahren zur Herstellung von 2-Fluorneplanocin A oder eines seiner pharmakologisch verträglichen Salze gemäß Anspruch 1 oder 2, wobei man bei dem Verfahren eine Verbindung der Formel

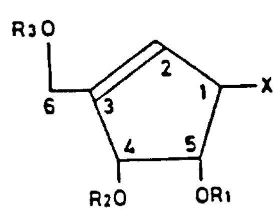

worin $R_1$ und $R_2$ jeweils Wasserstoff und eine Hydroxy-Schutzgruppe sind, $R_3$ eine Hydroxy-Schutz-

gruppe und X eine abspaltbare Gruppe ist, mit 2-Fluoradenin unter Herstellung einer Verbindung der folgenden Formel umsetzt:

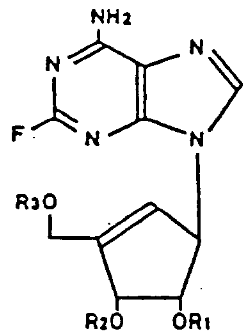

worin $R_1$, $R_2$ und $R_3$ die vorstehend angegebenen Bedeutungen besitzen, die Hydroxy-Schutzgruppen in ein Wasserstoffatom überführt und gegebenenfalls das derart gebildete 2-Fluorneplanocin A in eines seiner pharmakologisch verträglichen Salze überführt.

**4.** Verfahren nach Anspruch 3, wobei $R_1$ und $R_2$ jeweils eine Formyl-, Acetyl-, Methoxyacetyl, Benzoyl-, p-Chlorbenzyloxyacetyl-, t-Butyl-, Benzyl-, Trityl-, alpha-Ethoxyethyl-, alpha-Methoxyisopropyl-,Tetrahydropyranyl-, Methoxy-tetrahydropyranyl-, o-Nitrobenzyl- oder t-Butyldiphenylsilyl-Gruppe sind oder wobei $R_1$ und $R_2$ zusammen eine Isopropyliden-, Methoxymethylen-, Methoxyethyliden-, Ethoxymethylen-, Ethoxyethylen-, Benzyliden- oder Cycloalkyliden-Gruppe bilden.

**5.** Verfahren nach Anspruch 3 oder 4, wobei $R_3$ eine Formyl-, Acetyl-, Chloracetyl-, Trichloracetyl-, Trifluoracetyl-, Pivaloyl-, Benzoyl-, $\beta$-Benzoylpropionyl-, Phenoxyacetyl-, Trityloxyacetyl-, Trityl-, Monomethoxytrityl-, Dimethoxytrityl-, Trimethoxytrityl-, Monomethoxymethyl- oder Benzyl-Gruppe ist.

**6.** Verfahren nach einem der Ansprüche 3 bis 5, wobei X eine Methansulfonyloxy- oder p-Toluolsulfonyloxy-Gruppe oder ein Halogenatom ist.

**7.** Pharmazeutische Zusammensetzung, die 2-Fluorneplanocin A oder eines seiner pharmakologisch verträglichen Salze gemäß Anspruch 1 oder 2 und ein pharmazeutisch verträgliches Verdünnungsmittel umfaßt.

**8.** 2-Fluorneplanocin A oder eines seiner pharmakologisch verträglichen Salze gemäß Anspruch 1 oder 2 zur Anwendung in einem Verfahren zur Behandlung des menschlichen oder tierischen Körpers durch Therapie.

**9.** 2-Fluorneplanocin A oder eines seiner pharmakologisch verträglichen Salze gemäß Anspruch 1 oder 2 zur Anwendung als anti-neoplastisches Mittel.

**10.** Verwendung von 2-Fluorneplanocin A oder eines seiner pharmakologisch verträglichen Salze gemäß Anspruch 1 oder 2 bei der Herstellung eines Medikaments zur Behandlung eines Zustands, der ein anti-neoplastisches Mittel erfordert.

**Patentansprüche für folgende Vertragsstaaten : ES, GR**

**1.** Verfahren zur Herstellung von 2-Fluorneplanocin A oder eines seiner pharmakologisch verträglichen Salze, wobei man eine Verbindung der Formel:

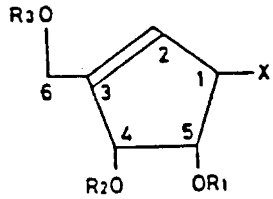

worin $R_1$ und $R_2$ jeweils ein Wasserstoffatom oder eine Hydroxy-Schutzgruppe sind, $R_3$ eine Hydroxy-Schutzgruppe ist und X eine abspaltbare Gruppe ist, mit 2-Fluoradenin unter Bildung einer Verbindung der folgenden Formel umsetzt:

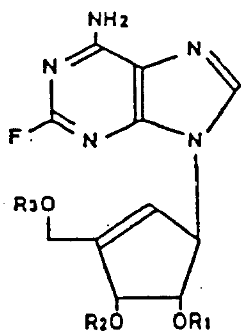

worin $R_1$, $R_2$ und $R_3$ die vorstehenden Bedeutungen besitzen, die Hydroxy-Schutzgruppen in Wasserstoffatome überführt und gegebenenfalls das derart gebildete 2-Fluorneplanocin A in eines seiner pharmakologisch verträglichen Salze überführt.

**2.** Verfahren nach Anspruch 1, wobei das Salz das Hydrochlorid, Sulfat, Phosphat, Acetat, Propionat, Tartrat, Zitrat, Glykolat, Glukonat, Succinat, Malat, Glutamat, Aspartat oder Methansulfonat ist.

**3.** Verfahren nach Anspruch 1 oder 2, wobei $R_1$ und $R_2$ jeweils eine Formyl-, Acetyl-, Methoxyacetyl-, Benzoyl-, p-Chlorbenzyloxyacetyl-, t-Butyl-, Benzyl-, Trityl-, alpha-Ethoxyethyl-, alpha-Methoxyisopropyl-,Tetrahydropyranyl-, Methoxy-tetrahydropyranyl-, o-Nitro-benzyl- oder t-Butyldiphenylsilyl-Gruppe sind oder wobei $R_1$ und $R_2$ zusammen eine Isopropyliden-, Methoxymethylen-, Methoxy-ethyliden-, Ethoxymethylen-, Ethoxyethylen-, Benzyliden- oder Cycloalkyliden-Gruppe bilden.

**4.** Verfahren nach einem der vorhergehenden Ansprüche, wobei $R_3$ eine Formyl-, Acetyl-, Chloracetyl-, Trichloracetyl-, Trifluoracetyl-, Pivaloyl-, Benzoyl-, $\beta$-Benzoylpropionyl-, Phenoxyacetyl-, Trityloxyacetyl-, Trityl-, Monomethoxytrityl-, Dimethoxytrityl-, Trimethoxytrityl-, Monomethoxymethyl- oder Benzyl-Gruppe ist.

**5.** Verfahren nach einem der vorhergehenden Ansprüche, wobei X eine Methansulfonyloxy- oder p-Toluolsulfonyloxy-Gruppe oder ein Halogenatom ist.

**6.** Verfahren zur Herstellung einer pharmazeutischen Zusammensetzung, bei dem man 2-Fluorneplanocin A oder eines seiner pharmakologisch verträglichen Salze gemäß Anspruch 1 oder 2 und ein pharmazeutisch verträgliches Verdünnungsmittel zusammenmischt.

**Revendications**

**Revendications pour les Etats contractants suivants : AT, BE, CH, DE, DK, FR, GB, IT, LI, LU, NL, SE**

**1.** 2-fluoronéplanocine A ou son sel pharmacologiquement acceptable.

**2.** 2-fluronéplanocine A selon la revendication 1 où le sel et le chlorhydrate, sulfate, phosphate, acétate, propionate, tartrate, citrate, glycolate, gluconate, succinate, malate, glutamate, aspartate ou méthanesulfonate.

3. Procédé de production de la 2-fluoronéplanocine A ou son sel pharmacologiquement acceptable selon la revendication 1 ou 2, lequel procédé comprend la réaction d'un composé de formule :

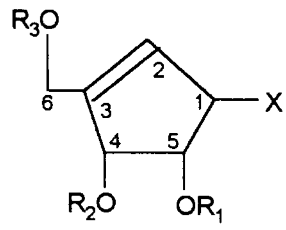

où $R_1$ et $R_2$ sont hydrogène ou un groupe hydroxy protecteur, $R_3$ est un groupe hydroxy protecteur et X est un groupe partant, avec de la 2-fluoroadénine pour donner un composé de la formule :

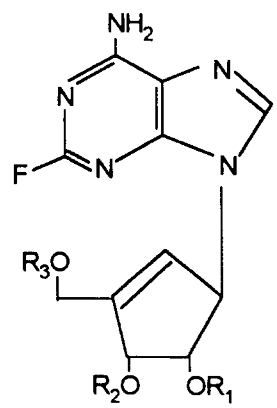

où $R_1$, $R_2$ et $R_3$ sont tels que définis ci-dessus, la conversion des groupes d'hydroxy protecteur en hydrogène et si on le souhaite la conversion de la 2-fluoronéplanocine A ainsi produite en son sel pharmacologiquement acceptable.

4. Procédé selon la revendication 3, où chacun de $R_1$ et $R_2$ est formyle, acétyle, méthoxyacétyle, benzoyle, p-chlorobenzyloxyacétyle, butyl tertiaire, benzyle, trityle, $\alpha$-éthoxyéthyle, $\alpha$-méthoxyisopropyle, tétrahydropyranyle, méthoxy-tétrahydropyranyle, o-nitro-benzyle ou t-butyldiphénylsilyle, ou bien où $R_1$ et $R_2$ forment ensemble un isopropylidène, un méthoxyméthylène, un méthoxyéthylidène, un éthoxyméthylène, un éthoxyéthylène, un benzylidène ou un cycloalkylidène.

5. Procédé selon la revendication 3 ou 4, où $R_3$ est un groupe formyle, acétyle, chloroacétyle, trichloroacétyle, trifluoroacétyle, pyvaloyle, benzoyle, $\beta$-benzoylpropionyle, phénoxyacétyle, trityloxyacétyle, trityle, monométhoxytrityle, diméthoxytrityle, triméthoxytrityle, monométhoxyméthyle ou benzyle.

6. Procédé selon l'une quelconque des revendications 3 à 5, où X est un groupe méthanesulfonyloxy ou p-toluénesulfonyloxy ou atome halogène.

7. Composition pharmaceutique qui comprend de la 2-fluoronéplanocine A ou son sel pharmacologiquement acceptable selon la revendication 1 ou 2 et un diluant pharmaceutiquement acceptable.

8. 2-fluoronéplanoncine A ou son sel acceptable pharmacologiquement selon la revendication 1 ou 2 à utiliser dans une méthode de traitement du corps humain ou animal par thérapie.

9. 2-fluoronéplanocine A ou son sel pharmacologiquement acceptable, tel que défini à la revendication 1 ou 2, pour une utilisation comme agent anti-néoplastique.

10. Utilisation de la 2-fluoronéplanocine A ou son sel pharmacologiquement acceptable selon la revendication 1 à 2 dans la fabrication d'un médicament pour le traitement d'une condition nécessitant un agent anti-néoplastique.

**Revendications pour les Etats contractants suivants : ES, GR**

1. Procédé de production de 2-fluoronéplanocine A ou son sel pharmacologiquement acceptable, lequel procédé comprend la réaction d'un composé de formule :

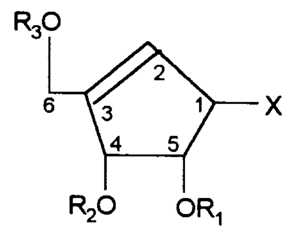

où $R_1$ et $R_2$ sont hydrogène ou un groupe hydroxy protecteur, $R_3$ est un groupe hydroxy protecteur et X est un groupe partant, avec de la 2-fluoroadénine pour donner un composé de la formule :

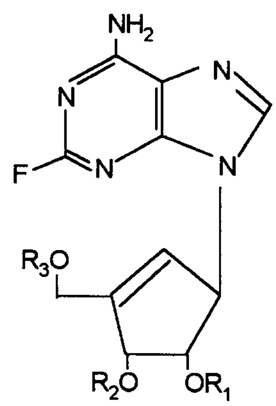

où $R_1$, $R_2$ et $R_3$ sont tels que définis ci-dessus, la conversion des groupes hydroxy protecteurs en hydrogène et, si on le souhaite, la conversion de la 2-fluoronéplanocine A ainsi produite en son sel pharmacologiquement acceptable.

2. Procédé selon la revendication 1 où le sel est le chlorhydrate, sulfate, phosphate, acétate, propionate, tartrate, citrate, glycolate, gluconate, succinate, malate, glutamate, aspartate ou méthanesulfonate.

3. Procédé selon la revendication 1 ou 2, où chacun de $R_1$ et $R_2$ est formyle, acétyle, méthoxyacétyle, benzoyle, p-chlorobenzyloxyacétyle, butyle tertiaire, benzyle, trityle, $\alpha$-éthoxyéthyle, $\alpha$-méthoxyisopropyle,tétrahydropyranyle, méthoxy-tétrahydropyranyle, o-nitro-benzyle ou t-butyldiphénylsilyle, ou bien où $R_1$ et $R_2$ forment ensemble un groupe isopropylidène, méthoxyméthylène, méthoxyéthylidène, éthoxyméthylène, éthoxyéthylène, benzylidène ou cycloalkylidène.

4. Procédé selon l'une quelconque des revendications précédentes où $R_3$ est un groupe formyle, acétyle, chloroacétyle, trichloroacétyle, trifluoroacétyle, pyvaloyle, benzoyle, $\beta$-benzoylpropionyle, phénoxyacétyle, trityloxyacétyle, trityle, monométhoxytrityle, diméthoxytrityle, triméthoxytrityle, monométhoxyméthyle ou benzyle.

5. Procédé selon l'une quelconque des revendications précédentes, où X est un groupe méthanesulfonyloxy ou p-toluenesulfonyloxy ou un atome halogène.

6. Procédé de préparation d'une composition pharmaceutique qui consiste à mélanger la 2-fluoronéplanocine A ou son sel pharmacologiquement acceptable selon la revendication 1 ou 2 et un diluant pharmaceutiquement acceptable.